# EUROPÄISCHE PATENTSCHRIFT

(11) **EP 1 171 096 B1**
(45) Veröffentlichungstag und Bekanntmachung des Hinweises auf die Patenterteilung: **21.12.2005**
(21) Anmeldenummer: 00918850.9
(22) Anmeldetag: 05.04.2000
(51) Int. Cl.: A61K 7/50

(54) **KOSMETISCHE ZUBEREITUNGEN**
COSMETIC PREPARATIONS
PREPARATIONS COSMETIQUES

(30) Priorität: 10.04.1999 DE 19916210
(43) Veröffentlichungstag der Anmeldung: 16.01.2002
(73) Patentinhaber: Cognis IP Management GmbH, 40589 Düsseldorf (DE)
(72) Erfinder: SCHMID, Karl, Heinz, D-40822 Mettmann (DE); FABRY, Bernd, D-41352 Korschenbroich (DE); HENSEN, Hermann, D-42781 Haan (DE); KOESTER, Josef, D-40221 Düsseldorf (DE)
(86) Internationale Anmeldenummer: PCT/EP2000/003015
(87) Internationale Veröffentlichungsnummer: WO 2000/061104

(56) Entgegenhaltungen:
- EP-A- 0 258 814
- EP-A- 0 510 564
- EP-A- 0 643 961
- WO-A-98/35652
- DE-A- 4 012 693
- DE-A- 4 108 626
- FR-A- 2 669 345
- FR-A- 2 785 798

## Beschreibung

### Gebiet der Erfindung

Die Erfindung befindet sich auf dem Gebiet der Kosmetik und betrifft Zubereitungen, die spezielle Ester von Hydroxycarbonsäuren zusammen mit Siliconverbindungen enthalten, sowie die Verwendung der Mischungen zur Herstellung von kosmetischen Zubereitungen.

### Stand der Technik

Seit Beginn der 60er Jahre haben Siliconverbindungen als Bestandteile kosmetischer Zubereitungen zunehmend an Bedeutung gewonnen, da sie das Haut- und Haargefühl dieser Mittel schon in kleinen Mengen verbessern, chemisch inert und mit praktisch allen kosmetischen Inhaltsstoffen kompatibel und schließlich aus dermatologischer Sicht unbedenklich sind. Problematisch ist jedoch in vielen Fällen, daß sich die Silicone nicht stabil in wäßrige Formulierungen einarbeiten lassen, was insbesondere bei längerer Lagerung oder Wärmeeinfluß zu unschönen Aufrahmungen oder gar irreversibler Phasentrennung führt. Dieses Problem tritt insbesondere dann auf, wenn die Emulsionen gleichzeitig noch Perlglanzwachse enthalten. Ein weiterer Nachteil im Zusammenhang mit dem Einsatz von Siliconverbindungen besteht darin, daß die Silicone eine Tendenz zeigen, sich auf Haut und Haaren anzureichern ("build-up-Effekt), was im Laufe der Zeit zu einem unerwünschten stumpfen Griff führt.

Demzufolge hat die Aufgabe der vorliegenden Erfindung darin bestanden, neue kosmetische Zubereitungen auf Basis von Siliconverbindungen zur Verfügung zu stellen, welche sich durch eine verbesserte Lager- und Temperaturbeständigkeit sowie einem verminderten build-up-Effekt auszeichnen.

### Beschreibung der Erfindung

Gegenstand der Erfindung sind kosmetische Zubereitungen, enthaltend
(a) Hydroxycarbonsäurealkyl- und/oder -alkenyloligoglykosidester und
(b) Siliconverbindungen.

Überraschenderweise wurde gefunden, daß die speziellen Hydroxycarbonsäureester in der Lage sind, Siliconverbindungen auch in höheren Konzentrationen und bei Wärmeeinfluß dauerhaft zu emulgieren. Gleichzeitig wird die Wasserbenetzbarkeit der Stoffe verbessert und damit die Anreicherung auf Haut und Haaren vermindert. Die Erfindung schließt die Erkenntnis mit ein, daß unter Einsatz der speziellen Hydroxycarbonsäurester auch stabile Emulsionen erhalten werden, die zusammen mit den Siliconverbindungen weiterhin Perlglanzwachse enthalten.

### Hydroxycarbonsäurealkyl- und/oder -alkenyloligoglykosidester

Ester von Hydroxycarbonsäuren und Alkyl- und Alkenyloligoglykosiden, die die Komponente (a) bilden, stellen bekannte nichtionische Tenside dar, die der Formel (I) folgen, 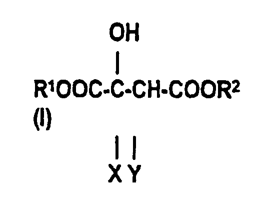 in der X für Wasserstoff oder eine CH₂COOR³-Gruppe, Y für Wasserstoff oder eine Hydroxylgruppe, R¹, R² und R³ unabhängig voneinander für Wasserstoff, ein Alkali- oder Erdalkalimetall, Ammonium, Alkylammonium, Hydroxyalkylammonium, Glucammonium oder den Rest eines Alkyl-und/oder Alkenyloligoglykosids der Formel (11) steht,

**R**^{**4**}**O-[G]**_{**p**} (II)

in der R⁴ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht, mit den Maßgaben, daß für den Fall, daß X für eine CH₂COOR³-Gruppe steht, Y Wasserstoff bedeutet und wenigstens einer der Reste R¹, R² und R³ für einen Glykosidrest steht. Die Herstellung und Verwendung dieser Stoffe als schaumstarke Tenside in kosmetischen Zubereitungen ist aus der europäischen Patentschrift **EP 0258814 B1** (Auschem) bekannt, auf die hier in vollem Umfang Bezug genommen werden soll.

Die Säurekomponente der nichtionischen Tenside kann sich von Äpfelsäure, Weinsäure oder Citronensäure sowie deren Gemischen ableiten. In Abhängigkeit der Zahl vorhandener Hydroxylgruppen in der Säurekomponente können Mono-, Di- oder Triester sowie deren technische Gemische vorliegen. Vorzugsweise gelangen jedoch Monoester bzw. Mischungen mit hohem Monoestergehalt zum Einsatz, welche vom Fachmann gemäß der Lehre der schon erwähnten EP 0258814 B1 erhalten werden können.

Die Alkyl- und/oder Alkenyloligoglykosidkomponente kann sich von Aldosen bzw. Ketosen mit 5 oder 6 Kohlenstoffatomen, vorzugsweise der Glucose ableiten. Die bevorzugten nichtionischen Tenside sind somit Ester von Alkyl- und/oder Alkenyloligoglucosiden. Die Indexzahl p in der allgemeinen Formel (II) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligo-glykosid eine analytisch ermittelte rechnerische Größe, die meistens eine gebrochene Zahl darstellt. Vorzugsweise werden Ester eingesetzt, deren Alkyl- - und/oder Alkenyloligoglykosidkomponente einen mittleren Oligomerisierungsgrad p von 1,1 bis 3,0 aufweist. Aus anwendungstechnischer Sicht sind solche Alkyl- und/oder Alkenyloligoglykosidester bevorzugt, deren Oligomerisierungsgrad kleiner als 1,7 ist und insbesondere zwischen 1,2 und 1,4 liegt. Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestem oder im Verlauf der Hydrierung von Aldehyden aus der Roelen'schen Oxosynthese erhalten werden. Bevorzugt sind C₉-C₁₁- bzw. C₈-C₁₀-Alkyloligoglucosidester mit einem DP = 1 bis 3, deren Alkylrest sich von entsprechenden Oxoalkoholen oder Alkoholen ableitet, die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können. Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucosidester auf Basis von gehärtetem C_{12/14}-Kokosalkohol mit einem DP von 1 bis 3. Derartige Produkte sind unter der Marke Eucarol® im Handel erhältlich.

Die Erfindung schließt die Erkenntnis ein, daß Mischungen von Hydroxycarbonsäurealkyl-und/oder alkenylglykosidestern und Alkyl- und/oder Alkenyloligoglykosiden zusammen mit Siliconverbindungen enthalten, besonders vorteilhafte anwendungstechnische Eigenschaften aufweisen. Solche temären Zubereitungen können im einfachsten Fall durch Vermischen der drei Bestandteile hergestellt werden. In einer besonderen Ausführungsform der Erfindung werden jedoch Siliconverbindungen zusammen mit Hydroxycarbonsäureglykosidestem eingesetzt, welche noch 1 bis 50, vorzugsweise 5 bis 25 und insbesondere 10 bis 15 Gew.-% unveresterte Glykoside enthalten.

### Siliconverbindungen

Siliconverbindungen, die die Gruppe (b) bilden, sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüchtig als auch nicht-flüchtig, flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil.** **91,** **27 (1976).**

Die Zubereitungen enthalten die Komponenten (a) und (b) im Gewichtsverhältnis 25 : 75 bis 75 : 25 und insbesondere 40 : 60 bis 60 : 40. In Summe sind die Mischungen der Komponenten (a) und (b) üblicherweise in den Zubereitungen in Mengen von 0,5 bis 20, vorzugsweise 1 bis 15 und insbesondere 2 bis 10 Gew.-% - bezogen auf die Mittel - enthalten.

### Gewerbliche Anwendbarkeit

Der Zusatz der speziellen Hydroxycarbonsäureester zu den Siliconverbindungen führt zu einer verbesserten Emulsionsstabilität und Wasserbenetzbarkeit. Ein weiterer Gegenstand der Erfindung betrifft daher die Verwendung der Mischungen zur Herstellung von kosmetischen Zubereitungen, in denen sie in Mengen von 0,5 bis 20, vorzugsweise 1 bis 15 und insbesondere 5 bis 10 Gew.-% - bezogen auf die Mittel - enthalten sein können.

### Kosmetische und/oder pharmazeutische Zubereitungen

Die erfindungsgemäßen Mischungen können zur Herstellung von kosmetischen Zubereitungen, wie beispielsweise Haarshampoos, Haarlotionen, Schaumbäder, Duschbäder, Cremes, Gele, Lotionen, alkoholische und wäßrig/alkoholische Lösungen, Emulsionen, Wachs/Fett-Massen, Stiftpräparaten, Pudem oder Salben dienen. Diese Mittel können ferner als weitere Hilfs- und Zusatzstoffe milde Tenside, Ölkörper, Emulgatoren, Überfettungsmittel, Perlglanzwachse, Konsistenzgeber, Verdickungsmittel, Polymere, Fette, Wachse, Stabilisatoren, biogene Wirkstoffe, Deowirkstoffe, Antischuppenmittel, Filmbildner, Quellmittel, UV-Lichtschutzfaktoren, Antioxidantien, Hydrotrope, Konservierungsmittel, Insektenrepellentien, Selbstbräuner, Solubilisatoren, Parfümöle, Farbstoffe, keimhemmende Mittel und dergleichen enthalten.

Typische Beispiele für geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, lsostearylstearat, I-sostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestem mit Polyolen, Siliconöle und/oder aliphatische bzw. naphthenische Kohlenwasserstoffe, wie z.B. Squalan, Squalen oder Dialkylcyclohexane in Betracht.

Als Emulgatoren kommen beispielsweise nichtionogene Tenside aus mindestens einer der folgenden Gruppen in Frage:
- (1): Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
- (2): C_{12/18}-Fettsäuremono- und -diester von Anlagerungsprodukten von 1 bis 30 Mol Ethylenoxid an Glycerin;
- (3): Glycerinmono- und -diester und Sorbitanmono- und -diester von gesättigten und ungesättigten Fettsäuren mit 6 bis 22 Kohlenstoffatomen und deren Ethylenoxidanlagerungsprodukte:
- (4): Alkylmono- und -oligoglycoside mit 8 bis 22 Kohlenstoffatomen im Alkylrest und deren ethoxylierte Analoga;
- (5): Anlagerungsprodukte von 15 bis 60 Mol Ethylenoxid an Ricinusöl und/oder gehärtetes Ricinusöl;
- (6): Polyol- und insbesondere Polyglycerinester, wie z.B. Polyglycerinpolyricinoleat, Polyglycerinpoly-12-hydroxystearat oder Polyglycerindimeratisostearat. Ebenfalls geeignet sind Gemische von Verbindungen aus mehreren dieser Substanzklassen;
- (7): Anlagerungsprodukte von 2 bis 15 Mol Ethylenoxid an Ricinusöl und/oder gehärtetes Ricinusöl;
- (8): Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
- (9): Mono-, Di- und Trialkylphosphate sowie Mono-, Di- und/oder Tri-PEG-alkylphosphate und deren Salze;
- (10): Wollwachsalkohole;
- (11): Polysiloxan-Polyalkyl-Polyether-Copolymere bzw. entsprechende Derivate;
- (12): Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin,
- (13): Polyalkylenglycole sowie
- (14): Glycerincarbonat.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den amphoteren bzw. zwitterionischen Emulgatoren kommen auch kationische Emulgatoren, insbesondere vom Esterquat-Typ in Frage.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether, Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

**Als Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quatemierte Hydroxyethylcellulose, die unter der Bezeichnung Polymer JR 400® von Amerchol erhältlich ist, kationische Stärke, Copolymere von Diallylammoniumsalzen und Acrylamiden, quatemierte Vinylpyrrolidon/Vinylimidazol-Polymere, wie z.B. Luviquat® (BASF), Kondensationsprodukte von Polyglycolen und Aminen, quatemierte Kollagenpolypeptide, wie beispielsweise Lauryldimonium hydroxypropyl hydrolyzed collagen (Lamequat®L/Grünau), quatemierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840 A** sowie deren vemetzte wasserlöslichen Polymere, kationische Chitinderivate wie beispielsweise quatemiertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quatemierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvemetzte und mit Polyolen vemetzte Polyacrylsäuren, Acrylamidopropyltrimethylammoniumchloridl Acrylat-Copolymere, Octylacrylamid/Methylmethacrylat/tert.Butylaminoethylmethacrylat/2-Hydroxyproylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

Typische Beispiele für **Fette** sind Glyceride, als **Wachse** kommen u.a. natürliche Wachse, wie z.B. Candelillawachs, Camaubawachs, Japanwachs, Espartograswachs, Korkwachs, Guarumawachs, Reis-keimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.

Als **Stabilisatoren** können Metallsalze von Fettsäuren, wie z.B. Magnesium-, Aluminium- und/oder Zinkstearat bzw. -ricinoleat eingesetzt werden.

Unter **biogenen Wirkstoffen** sind beispielsweise Tocopherol, Tocopherolacetat, Tocopherolpalmitat, Ascorbinsäure, Desoxyribonudeinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Als **Deowirkstoffe** kommen z.B. Antiperspirantien wie etwa Aluminiumchlorhydate in Frage. Hierbei handelt es sich um farblose, hygroskopische Kristalle, die an der Luft leicht zerfließen und beim Eindampfen wäßriger Aluminiumchloridlösungen anfallen. Aluminiumchlorhydrat wird zur Herstellung von schweißhemmenden und desodorierenden Zubereitungen eingesetzt und wirkt wahrscheinlich über den partiellen Verschluß der Schweißdrüsen durch Eiweiß- und/oder Polysaccharidfällung [vgl. **J.Soc. Cosm.Chem.** **24,** **281 (1973)].** Unter der Marke Locron® der Hoechst AG, Frankfurt/FRG, befindet beispielsweise sich ein Aluminiumchlorhydrat im Handel, das der Formel [Al₂(OH)₅Cl]*2,5 H₂O entspricht und dessen Einsatz besonders bevorzugt ist [vgl. **J.Pharm.Pharmacol.** **26,** **531 (1975)].** Neben den Chlorhydraten können auch Aluminiumhydroxylactate sowie saure Aluminium/Zirkoniumsalze eingesetzt werden. Als weitere Deowirkstoffe können Esteraseinhibitoren zugesetzt werden. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut soweit absenkt, daß dadurch die Enzyme inhibiert werden. Weitere Stoffe, die als Esteraseinhibitoren in Betracht kommen, sind Sterolsulfate oder-phosphate, wie beispielsweise Lanosterin-, Cholesterin-, Campesterin-, Stigmasterin- und Sitosterinsulfat bzw. -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester. Antibakterielle Wirkstoffe, die die Keimflora beeinflussen und schweißzersetzende Bakterien abtöten bzw. in ihrem Wachstum hemmen, können ebenfalls in den Stiftzubereitungen enthalten sein. Beispiele hierfür sind Chitosan, Phenoxyethanol und Chlorhexidingluconat. Besonders wirkungsvoll hat sich auch 5-Chlor-2-(2,4-dichlorphen-oxy)-phenol erwiesen, das unter der Marke Irgasan® von der Ciba-Geigy, Basel/CH vertrieben wird.

Als **Antischuppenmittel** können Climbazol, Octopirox und Zinkpyrethion eingesetzt werden. Gebräuchliche **Filmbildner** sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quatemäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil.** **108****, 95 (1993)** entnommen werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher wie in der **EP 0693471 B1** beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben;
- Propan-1,3-dione, wie z.B. 1-(4-tert.Butylphenyl)-3-(4'methoxyphenyl)propan-1,3-dion;
- Ketotricyclo(5.2.1.0)decan-Derivate, wie in der **EP 0694521 B1** beschrieben.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bomylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro-oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyt-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner **Hydrotrope**, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyl-m-toluamid, 1,2-Pentandiol oder Insekten-Repellent 3535 in Frage, als Selbstbräuner eignet sich Dihydroxyaceton.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättem (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzem (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax).

Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethem zählen beispielsweise Benzytethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexyisalicylat, Vertofix Coeur, lso-E-Super, Fixolide NP, Evemyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.%, bezogen auf die gesamte Mischung, eingesetzt.

Typische Beispiele für keimhemmende **Mittel** sind Konservierungsmittel mit spezifischer Wirkung gegen gram-positive Bakterien wie etwa 2,4,4'-Trichlor-2'-hydroxydiphenylether, Chlorhexidin (1,6-Di-(4-chlorphenyl-biguanido)-hexan) oder TCC (3,4,4'-Trichlorcarbanilid). Auch zahlreiche Riechstoffe und etherische Öle weisen antimikrobielie Eigenschaften auf. Typische Beispiele sind die Wirkstoffe Eugenol, Menthol und Thymol in Nelken-, Minz- und Thymianöl. Ein interessantes natürliches Deomittel ist der Terpenalkohol Farnesol (3,7,11-Trimethyl-2,6,10-dodecatrien-1-ol), der im Lindenblütenöl vorhanden ist und einen Maiglöckchengeruch hat. Auch Glycerinmonolaurat hat sich als Bakteriostatikum bewährt. Üblicherweise liegt der Anteil der zusätzlichen keimhemmenden Mittel bei etwa 0,1 bis 2 Gew.% - bezogen auf den Feststoffanteil der Zubereitungen.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

Die Stabilität von silicon- und ggf. auch perlglanzhaltigen Emulsionen unter Einsatz verschiedener Emulgatoren wurde bei Lagerung über einen Zeitraum von 1 bis 4 Wochen bei 20 bis 40 °C untersucht. Dabei bedeutet (+) stabil und (-) Phasentrennung bzw. Sedimentation. Die Ergebnisse sind in Tabelle 1 zusammengefaßt. Die Beispiele 1 bis 6 sind erfindungsgemäß, die Beispiele V1 und V2 dienen zum Vergleich. Alle Mengenangaben verstehen sich als Gew.-%.

**Tabelle 1**

| **Emulsionsstabilität** | | | | | | | | |
|---|---|---|---|---|---|---|---|---|
| **Zusammensetzung** | **1** | **2** | **3** | **4** | **5** | **6** | **V1** | **V2** |
| Sodium Laurylglucoside Citrate | 2,5 | - | - | 2,5 | - | - | - | - |
| Sodium Laurylglucoside Malate | - | 2,5 | - | - | 2,5 | - | - | - |
| Sodium Cocoylglucoside Tartrate | - | - | 2,5 | - | - | 2,5 | - | - |
| Coco Glucosides | - | - | - | - | - | - | 2,5 | 2,5 |
| Ethylenglycol Distearate | - | - | - | 3,0 | 3,0 | 3,0 | - | 3,0 |
| Dimethicone | 1,0 | - | - | 1,0 | - | - | 1,0 | 1,0 |
| Cyclodimethicone | - | 1,0 | - | - | 1,0 | - | - | - |
| Amodimethicone | - | - | 1,0 | - | - | 1,0 | - | - |
| Wasser | ad 100,0 | | | | | | | |

| ***Emulsionsstabilität*** | | | | | | | | |
|---|---|---|---|---|---|---|---|---|
| -1 Woche, 20 °C | + | + | + | + | + | + | + | - |
| - 2 Wochen, 20 °C | + | + | + | + | + | + | + | - |
| - 4 Wochen, 20°C | + | + | + | + | + | + | + | - |
| -1 Woche, 40 °C | + | + | + | + | + | + | - | - |
| - 2 Wochen, 40 °C | + | + | + | + | + | + | - | - |
| - 4 Wochen, 40 °C | + | + | + | + | + | + | - | - |

## Patentansprüche

1. Kosmetische und/oder pharmazeutische Zubereitungen, enthaltend
(a) Hydroxycarbonsäurealkyl- und/oder -alkenyloligoglykosidester und
(b) Siliconverbindungen.
mit der Maßgabe, dass die Komponenten (a) zu (b) im Gewichtsverhältnis 25 : 75 bis 75 : 25 vorliegen.

2. Zubereitungen nach Anspruch 1, **dadurch gekennzeichnet, daß** sie als Komponente (a) Hydroxycarbonsäurealkyl- und/oder -alkenyloligoglykosidester der Formel (I) enthalten, 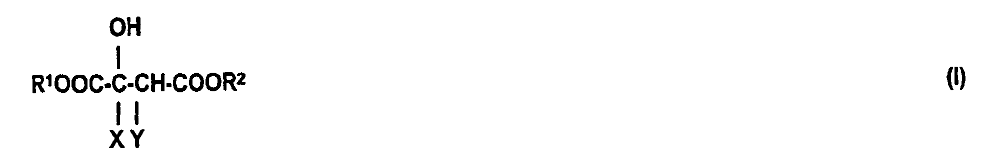 in der X für Wasserstoff oder eine CH₂COOR³-Gruppe, Y für Wasserstoff oder eine Hydroxylgruppe, R¹, R² und R³ unabhängig voneinander für Wasserstoff, ein Alkali- oder Erdalkalimetall, Ammonium, Alkylammonium, Hydroxyalkylammonium, Glucammonium oder den Rest eines Alkyl- und/oder Alkenyloligoglykosids der Formel (II) steht,
**R**^{**4**}**O-[G]**_{**p**} **(II)**
in der R⁴ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht, mit den Maßgaben , daß für den Fall, daß X für eine CH₂COOR³-Gruppe steht, Y Wasserstoff bedeutet und wenigstens einer der Reste R¹, R² und R³ für einen Glykosidrest steht.

3. Zubereitungen nach den Ansprüchen 1 und/oder 2, **dadurch gekennzeichnet, daß** sie Ester von Äpfelsäure, Weinsäure und/oder Citronensäure mit Alkyl- und/oder Alkenyloligoglykosiden enthalten.

4. Zubereitungen nach mindestens einem der Ansprüche 1 bis 3, **dadurch gekennzeichnet, daß** sie Ester enthalten, welche noch 1 bis 50 Gew.-% unveresterte Glykoside enthalten.

5. Zubereitungen nach mindestens einem der Ansprüche 1 bis 4, **dadurch gekennzeichnet, daß** sie als Komponente (b) flüchtige Siliconverbindungen enthalten.

6. Zubereitungen nach mindestens einem der Ansprüche 1 bis 4, **dadurch gekennzeichnet, daß** sie als Komponente (b) nicht-flüchtige Siliconverbindungen enthalten.

7. Zubereitungen nach mindestens einem der Ansprüche 1 bis 6, **dadurch gekennzeichnet, daß** sie Siliconverbindungen enthalten, die ausgewählt sind aus der Gruppe, die gebildet wird von Dimethylpolysiloxanen, Methylphenylpolysiloxanen, Simethiconen, cyclischen Siliconen sowie amino-, fettsäure-, alkohol-, polyether-, epoxy-, fluor-, glykosid- und/oder alkylmodifizierte Siliconverbindungen.

8. Zubereitungen nach mindestens einem der Ansprüche 1 bis 7, **dadurch gekennzeichnet, daß** sie die Mischungen der Komponenten (a) und (b) in Summe in Mengen von 0,5 bis 20 Gew.-% - bezogen auf die Mittel - enthalten.

9. Verwendung von Mischungen, enthaltend
(a) Hydroxycarbonsäurealkyl- und/oder -alkenyloligoglykosidester und
(b) Siliconverbindungen
mit der Maßgabe, dass die Komponenten (a) zu (b) im Gewichtsverhältnis 25 : 75 bis 75 : 25 vorliegen zur Herstellung von kosmetischen Zubereitungen.

## Claims

1. Cosmetic and/or pharmaceutical preparations containing
(a) hydroxycarboxylic acid alkyl and/or alkenyl oligoglycoside esters and
(b) silicone compounds,
with the proviso that they contain components (a) and (b) in a ratio by weight of 25:75 to 75:25.

2. Preparations as claimed in claim 1, **characterized in that** they contain as component (a) hydroxycarboxylic acid alkyl and/or alkenyl oligoglycoside esters corresponding to formula (I): 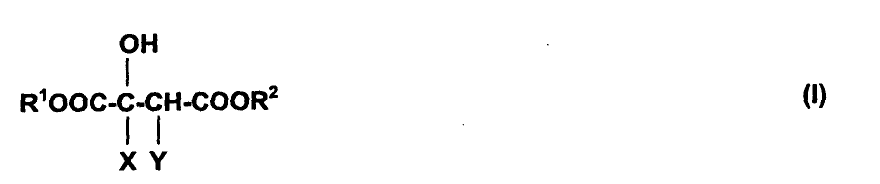 where X is hydrogen or a CH₂COOR³ group, Y is hydrogen or a hydroxyl group, R¹, R² and R³ independently of one another represent hydrogen, an alkali metal or alkaline earth metal, ammonium, alkyl ammonium, hydroxyalkyl ammonium, glucammonium or the residue of an alkyl and/or alkenyl oligoglycoside corresponding to formula **(II):**
**R**^{**4**}**O-[G]**_{**p**} **(II)**
in which R⁴ is a C₄₋₂₂ alkyl and/or alkenyl group, G is a sugar unit containing 5 or 6 carbon atoms and p is a number of 1 to 10, with the provisos that, where X is a CH₂COOR³ group, Y is hydrogen and at least one of the substituents R¹, R² and R³ is a glycoside unit.

3. Preparations as claimed in claims 1 and/or 2, **characterized in that** they contain esters of malic acid, tartaric acid and/or citric acid with alkyl and/or alkenyl oligoglycosides.

4. Preparations as claimed in at least one of claims 1 to 3,
**characterized in that** they contain esters which still contain 1 to 50% by weight of unesterified glycosides.

5. Preparations as claimed in at least one of claims 1 to 4, **characterized in that** they contain volatile silicone compounds as component (b).

6. Preparations as claimed in at least one of claims 1 to 4, **characterized in that** they contain non-volatile silicone compounds as component (b).

7. Preparations as claimed in at least one of claims 1 to 6, **characterized in that** they contain silicone compounds selected from the group consisting of dimethyl polysiloxanes, methyl phenyl polysiloxanes, simethicones, cyclic silicones and amino-, fatty acid-, alcohol-, polyether-, epoxy-, fluorine-, glycoside- and/or alkyl-modified silicone compounds.

8. Preparations as claimed in at least one of claims 1 to 7, **characterized in that** they contain the mixtures of components (a) and (b) in total quantities of 0.5 to 20% by weight, based on the preparation.

9. The use of mixtures containing
(a) hydroxycarboxylic acid alkyl and/or alkenyl oligoglycoside esters and
(b) silicone compounds,
with the proviso that components (a) and (b) are present in a ratio by weight of 25:75 to 75:25,
for the production of cosmetic preparations.

## Revendications

1. Préparations cosmétiques et/ou pharmaceutiques contenant
(a) des alkyl- et/ou alcényloligoglycoside esters d'acide hydroxycarboxylique et
(b) des composés de silicone,
étant précisé que le rapport pondéral entre les composants (a) et (b) est de 25:75 à 75:25.

2. Préparations selon la revendication 1,
**caractérisées en ce que**
comme composant (a), elles contiennent, des alkyl- et/ou alcényloligoglycoside esters d'acide hydroxycarboxylique de formule (I), 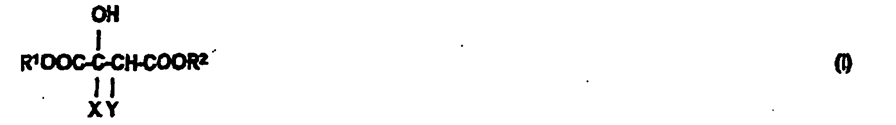 dans laquelle X représente un atome d'hydrogène ou un groupe CH₂COOR³ ; Y est un atome d'hydrogène ou un groupe hydroxyle ; R¹, R², R³, sont indépendamment les uns des autres, un atome d'hydrogène, un métal alcalin ou alcalino-terreux, de l'ammonium, de l'alkylammonium, de l'hydroxyalkylammonium, du glucammonium ou le radical d'un alkyl- et/ou alcényl oligoglycoside de formule (II),
**R**^{**4**}**O-[G]**_{**p**} **(II)**
dans laquelle R⁴ représente un radical alkyle et/ou alcényle portant 4 à 22 atomes de carbone, G est un radical sucre portant 5 ou 6 atomes de carbone, et p est un nombre de 1 à 10, étant précisé que dans le cas où X représente un groupe CH₂COOR³, Y représente un atome d'hydrogène et au moins l'un des radicaux R¹, R² et R³ représente un radical glycoside.

3. Préparations selon les revendications 1 et/ou 2,
**caractérisées en ce qu'**
elles contiennent des esters de l'acide malique, de l'acide tartrique et/ou de l'acide citrique avec des alkyl-et/ou alcényl oligoglycosides.

4. Préparations selon au moins l'une des revendications 1 à 3,
**caractérisées en ce qu'**
elles contiennent des esters qui renferment encore 1 à 50 % en poids de glycosides non estérifiés.

5. Préparations selon au moins l'une des revendications 1 à 4,
**caractérisées en ce qu'**
elles contiennent des composés de silicone volatils en tant que composant (b).

6. Préparations selon au moins l'une des revendications 1 à 4,
**caractérisées en ce qu'**
elles contiennent des composés de silicone non volatils en tant que composant (b).

7. Préparations selon au moins l'une des revendications 1 à 6,
**caractérisées en ce qu'**
elles contiennent des composés de silicone choisis dans le groupe formé de diméthylpolysiloxanes, de méthylphénylpolysiloxanes, de siméthicones, de silicones cycliques ainsi que de composés de silicone modifiés par un groupe amino, acide gras, alcool, polyéther, époxy, fluor, glycoside et/ou alkyle.

8. Préparations selon au moins l'une des revendications 1 à 7,
**caractérisées en ce qu'**
elles contiennent les mélanges des composants (a) et (b) dans des quantités totales de 0,5 à 20 % en poids par rapport aux agents.

9. Utilisation de mélanges contenant
(a) des alkyl- et/ou alcényloligoglycoside esters d'acide hydroxycarboxylique et
(b) des composés de silicone,
étant précisé que le rapport pondéral entre les composants (a) et (b) est de 25:75 à 75:25, pour l'obtention de préparations cosmétiques.
